# DEMANDE DE BREVET EUROPEEN

(11) **EP 1 364 641 A1**
(43) Date de publication de la demande: **26.11.2003**
(21) Numéro de dépôt: 03291078.8
(22) Date de dépôt: 05.05.2003
(51) Int. Cl.: A61K 7/48, A61K 7/075, A61K 31/315, A61P 17/06, A61P 17/10

(54) **Utilisation d'au moins un complexe de métal comme desquamant**

(30) Priorité: 13.05.2002 FR 0205855
(71) Demandeur: L'OREAL, 75008 Paris (FR)
(72) Inventeur: Galey, Jean-Baptiste, 93600 Aulnay-Sous-Bois (FR)
(74) Mandataire: Allab, Myriam

(57) **Abrégé**

L'invention se rapporte à l'utilisation cosmétique d'au moins un complexe de métal dans une composition cosmétique comprenant un milieu physiologiquement acceptable, en tant qu'agent destiné à favoriser la desquamation de la peau et/ou à stimuler le renouvellement épidermique.

Le complexe de métal selon l'invention peut ainsi être utilisé à des fins cosmétiques, pour prévenir ou traiter la peau sèche et/ou les signes cutanés du vieillissement et/ou la pigmentation de la peau et/ou la peau grasse à tendance acnéique. En variante, il peut être utilisé à titre de médicament, en particulier pour préparer une composition destinée à prévenir ou traiter les désordres cutanés liés aux hyperkératoses.

## Description

L'invention se rapporte à l'utilisation cosmétique d'au moins un complexe de métal dans une composition cosmétique comprenant un milieu physiologiquement acceptable, en tant qu'agent destiné à favoriser la desquamation de la peau et/ou à stimuler le renouvellement épidermique.

Le complexe de métal selon l'invention peut ainsi être utilisé à des fins cosmétiques, pour prévenir ou traiter la peau sèche et/ou les signes cutanés du vieillissement et/ou la pigmentation de la peau et/ou la peau grasse à tendance acnéique. En variante, il peut être utilisé à titre de médicament, en particulier pour préparer une composition destinée à prévenir ou traiter les désordres cutanés liés aux hyperkératoses.

La desquamation est un phénomène naturel lié au fait que l'épiderme, qui constitue la couche supérieure de la peau, est en constante régénération.

L'épiderme humain est constitué de plusieurs assises de cellules dans lesquelles on trouve principalement quatre types de cellules: les kératinocytes, très majoritaires, les mélanocytes, les cellules de Langerhans et les cellules de Merkel. La répartition de ces cellules en plusieurs couches superposées explique le caractère stratifié de l'épiderme.
L'épiderme est conventionnellement divisé en une couche basale de kératinocytes qui constitue la couche germinative de l'épiderme, une couche dite épineuse constituée de plusieurs couches de cellules polyédriques disposées sur les cellules germinatives, une couche dite granuleuse constituée de cellules aplaties contenant des inclusions cytoplasmiques distinctes, les grains de kératohyaline, et enfin une couche supérieure appelée couche cornée (ou stratum corneum), constituée de kératinocytes au stade terminal de leur différenciation appelés cornéocytes. Les cornéocytes sont des cellules momifiées, anucléées qui dérivent des kératinocytes et s'éliminent par desquamation. Cette perte en surface est compensée par la migration de cellules de l'assise basale vers la surface de l'épiderme. Il s'agit du renouvellement perpétuel de l'épiderme. Une élimination forcée de la couche cornée accélère le renouvellement et permet de lutter contre le vieillissement cutané.

Les cornéocytes sont principalement composés d'une matrice fibreuse contenant des cytokératines, entourée d'une structure très résistante de 15 nm d'épaisseur, appelée enveloppe cornée ou cornifiée. L'empilement de ces cornéocytes constitue la couche cornée qui est responsable de la fonction de barrière de l'épiderme. Au cours du processus normal de desquamation, les cornéocytes les plus superficiels se détachent de la surface de l'épiderme.
Des structures intercellulaires dérivant des desmosomes, appelées cornéosomes ou cornéodesmosomes, ont été décrites dans la couche cornée. Des études récentes ont montré leur importance majeure dans la cohésion intercornéocytaire ainsi que dans le processus de desquamation.
La cornéodesmosine, par ailleurs caractérisée dans la demande EP-A-0 972 042 de la Demanderesse, est une protéine de la couche cornée de l'épiderme, impliquée dans la cohésion intercornéocytaire et constitutive des cornéodesmosomes.
Dans la couche cornée, une corrélation étroite existe entre la dissociation cellulaire et la protéolyse de certains composants cornéodesmosomaux comme la desmogléine I et la cornéodesmosine. Plusieurs protéases à sérine de type trypsine ou chymotrypsine semblent être impliquées dans la protéolyse des cornéodesmosomes, comme en particulier des protéases de type Chymotrypsine-like ou Trypsine-like (Lundström A., Egelrud T., The Journal of Investigative Dermatology; 1988, 91:340-343 et 1990, 84:216-220).

Le vieillissement cutané résulte de deux processus distincts et indépendants qui font intervenir des facteurs intrinsèques ou extrinsèques.
Le vieillissement intrinsèque ou chronobiologique correspond au vieillissement « normal » ou physiologique lié à l'age.
Le vieillissement extrinsèque correspond au vieillissement provoqué d'une manière générale par l'environnement et plus particulièrement au photovieillissement dû à l'exposition au soleil, à la lumière ou à tout autre rayonnement (EP-A2-0 815 840, Kligman, A. M. *et al*., Journal of Cutaneous Aging and Cosmetic Dermatology, Vol. 1, N°.1, pp. 5-12 (1988)).

La présente invention s'intéresse notamment au vieillissement cutané intrinsèque ou physiologique ainsi qu'au vieillissement cutané extrinsèque.

Le vieillissement cutané en général se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté ou par l'apparition de télangiectasies.

Les changements de la peau dus au vieillissement intrinsèque sont la conséquence d'une sénescence génétiquement programmée où interviennent des facteurs endogènes. Ce vieillissement intrinsèque se traduit notamment par un ralentissement du renouvellement des cellules de l'épiderme et l'apparition de fines rides ou ridules.

Au contraire, le vieillissement extrinsèque résulte, au niveau du derme, de la dégradation des fibres de collagène ayant notamment pour conséquence des altérations cliniques telles que des rides épaisses et la formation d'une peau molle et tannée.

Certains agents cosmétiques favorisent la desquamation, c'est-à-dire l'élimination des cellules « mortes » situées à la surface de la couche cornée de l'épiderme.
C'est en particulier le cas des α-hydroxy acides (AHA) comme l'acide lactique ou l'acide glycolique ou des β-hydroxyacides (BHA) comme l'acide salicylique qui induisent, par application topique à des concentrations de quelques % une desquamation visible après quelques jours. Leur mode d'action n'est pas connu en détail mais est en partie lié à l'acidification qu'ils induisent au niveau des différentes couches de l'épiderme. Leur effet desquamant n'est observé que si le pH des produits appliqués est inférieur à 3 Plus précisément, l'action des AHA semble liée à la destruction du corps protéique du cornéosome, ce qui se traduit par une accélération de la séparation des cornéocytes
Alors que l'acide lactique et l'acide salicylique accélèrent le détachement des cornéocytes de manière brutale et non spécifique, l'acide 5-octanoyl salicylique semblent avoir un mode d'action plus ciblé, permettant un « découpage » plus propre au niveau de l'interface *compactum* / *disjonctum*, probablement du fait de son caractère lipophile lui permettant une pénétration via les couches de lipides intercellulaires.

Dans tous les cas, face à cette agression, l'épiderme réagit immédiatement par une réponse physiologique qui se traduit par une irritation locale et par une accélération du turnover épidermique afin de rétablir l'épaisseur et les fonctions du stratum corneum.

Le problème que vise à résoudre la présente invention consiste donc à trouver de nouveaux composés prodesquamants ne possédant pas les effets secondaires irritants des AHA et des autres agents kératolytiques connus.

En outre, le désir de conserver une apparence jeune conduit toujours à la recherche incessante de nouveaux composés et/ou de nouvelles compositions permettant de maintenir ou d'améliorer l'apparence de la peau.

La Demanderesse a maintenant découvert de manière surprenante et inattendue que certains complexes de métaux présentant des propriétés hydrolytiques trouvaient à ce titre diverses applications en cosmétique et en dermatologie.

Certains complexes de métaux sont déjà connus pour leurs propriétés hydrolytiques (voir par exemple Chin, *Acc. Chem. Res.* 1991, 24 : 145).
Il est connu également du document EP 0 820 763 B1 l'utilisation de complexes métalliques (ZnCl₂ ou CaCl₂,) de dérivés de l'acide N,N'-dibenzyl éthylènediamine N,N'-diacétique comme agents dépigmentants de la peau humaine ainsi qu'un procédé de dépigmentation et/ou de blanchiment de la peau colorée ou tachée.

En revanche, il n'a jamais été décrit dans l'art antérieur que les complexes de métaux constituent d'excellents actifs cosmétiques sélectifs et non irritants capables de favoriser la desquamation et/ou stimuler le renouvellement épidermique et par conséquent trouver des applications dans le traitement des signes cutanés du vieillissement de la peau, de la peau sèche, de l'hyperpigmentation, de la peau grasse à tendance acnéique et de tous les désordres cutanés engendrés par les hyperkératoses.

La présente invention a donc pour objet l'utilisation cosmétique d'au moins un complexe de métal dans une composition cosmétique comprenant un milieu physiologiquement acceptable, en tant qu'agent destiné à favoriser la desquamation de la peau et/ou à stimuler le renouvellement épidermique. Avantageusement, le dit complexe de métal est un agent cosmétique qui contribue à lutter contre la peau sèche et/ou les signes cutanés du vieillissement et/ou la peau grasse à tendance acnéique.

Les complexes de métaux suivant l'invention peuvent ainsi être utilisés à des fins cosmétiques, dans une composition cosmétique, en tant qu'agent cosmétique pour prévenir ou traiter la peau sèche et/ou les signes cutanés du vieillissement et/ou la peau grasse à tendance acnéique. L'invention a donc également pour objet ces utilisations cosmétiques des complexes de métaux.

Elle a encore pour objet un procédé de traitement cosmétique de la peau sèche et/ou des signes cutanés du vieillissement et/ou de la peau grasse à tendance acnéique, comprenant l'application topique sur la peau d'une composition comprenant, dans un milieu physiologiquement acceptable, d'au moins un complexe de métal suivant l'invention.

Elle a encore pour objet un procédé de traitement cosmétique pour diminuer la pigmentation de la peau comprenant l'application topique sur la peau d'une composition comprenant, dans un milieu physiologiquement acceptable, au moins un complexe de métal décrit dans la revendication 5.

L'invention se rapporte encore à un procédé de traitement cosmétique destiné à favoriser la desquamation et/ou stimuler le renouvellement épidermique, caractérisé en ce qu'il comprend l'application topique sur la peau d'une composition comprenant, dans un milieu physiologiquement acceptable, une quantité cosmétiquement acceptable d'au moins un complexe de métal suivant l'invention pour favoriser ladite desquamation et/ou stimulation. Avantageusement, le procédé de traitement cosmétique est adapté à une peau choisi parmi: une peau sèche, une peau qui présente des signes cutanés du vieillissement, une peau qui présente une hyperpigmentation ou une peau grasse à tendance acnéique.

En variante, le complexe de métal peut être utilisé à titre de médicament, en particulier pour préparer une composition pharmaceutique ou dermatologique comprenant un milieu physiologiquement acceptable, la dite composition étant destinée à prévenir ou traiter les désordres cutanés liés aux hyperkératoses, tels que le parapsoriasis, le psoriasis, l'ichtyose, le lichen.

Par milieu physiologiquement acceptable, on comprend un milieu compatible avec la peau, les muqueuses, les ongles, le cuir chevelu et les cheveux.

Les complexes de métaux suivant l'invention peuvent éventuellement prendre des formes différentes une fois solubilisés dans différents solvants comme l'eau, notamment se dimériser en complexes binucléaires ou s'oligomériser en complexes polynucléaires, tout en conservant leurs propriétés.

Les complexes de métaux au sens de la présente invention sont caractérisés par une activité hydrolytique spécifique ; la dite activité hydrolytique est évaluée pour sa capacité à accélérer d'au moins un facteur 2 l'hydrolyse en solution aqueuse à pH neutre ou légèrement alcalin, ou dans un mélange eau-acétonitrile ou eau-acétone, d'un substrat choisi parmi le phosphate de p-nitro phénol, l'acétate de p-nitro phénol, l'acétate de p-nitro aniline.

Cette méthode d'évaluation est une adaptation de travaux décrits entre autres par Kimura et coll. (*J. Am. Chem. Soc.* 1990, 112 :5805).
Cette méthode est basée sur le suivi en fonction du temps de l'hydrolyse d'un substrat modèle : le phosphate de p-nitrophénol, l'acétate de p-nitrophénol ou l'acétate de p-nitroaniline à pH neutre ou légèrement alcalin en solution aqueuse ou dans un mélange eau-acétone ou eau-acétonitrile comprenant avantageusement 10 à 50% d'acétone ou d'acétonitrile, préférentiellement 20%, pour permettre la solubilisation de certains complexes ou substrats,.
Cette méthode consiste essentiellement à ajouter successivement dans une cuvette UV :
- un tampon, avantageusement un tampon tris, phosphate ou borate, préférentiellement un tampon tris, à une concentration comprise entre 10 et 100 mM, avantageusement 50 mM, à pH compris entre 7 et 9, avantageusement à pH 8,
- une solution comprise entre 10⁻⁵ et 10⁻² M, avantageusement 10 mM en complexe de métal suivant l'invention dans l'eau ou dans un mélange eau-acétonitrile ou eau-acétone et,
- une solution de substrat modèle à une concentration comprise entre 2 et 20 mM, avantageusement à 10 mM dans l'acétonitrile ou l'acétone.

On suit alors l'augmentation de la densité optique à 410 nm à une température comprise entre 30 et 50°C, avantageusement entre 35 et 40°C, préférentiellement à 37°C en fonction du temps pendant 1 heure.
L'activité hydrolytique du complexe est déterminée par le rapport entre la pente de la droite obtenue en présence de complexe et celle de la droite obtenue dans une expérience témoin sans complexe, qui mesure l'hydrolyse spontanée du substrat.

Ainsi, les complexes de métaux suivant la présente invention sont ceux qui accélèrent d'au moins un facteur 2 l'hydrolyse d'un substrat choisi parmi le phosphate de p-nitrophénol, l'acétate de p-nitrophénol ou l'acétate de p-nitroaniline, à pH neutre ou légèrement alcalin, en solution aqueuse ou dans un mélange constitué d'eau et d'acétonitrile ou d'eau et d'acétone.

Avantageusement les complexes de métaux suivant l'invention répondent à la formule (I) suivante:

(I) Lₚ-Mⁿ⁺

dans laquelle,
- M représente un métal choisi par le zinc, le cobalt, le cuivre, le fer, le lanthane, le palladium, le manganèse, le molybdène, l'europium, le cérium ou le zirconium;
- n désigne un nombre choisi parmi 2, 3 ou 4 ;
- L représente un agent complexant choisi parmi un bidentate, un tridentate, un tétradentate, un pentadentate ou hexadentate ;
- p désigne un nombre choisi parmi 1, 2 ou 3.

L'invention concerne également les isomères optiques et/ou géométriques des complexes de métaux précédemment décrits, seuls ou en mélange en toutes proportions, ainsi que les sels physiologiquement acceptables de ces dérivés.

Par agent complexant bidentate, tridentate, tétradentate, pentadentate ou hexadentate, on entend les agents classiquement utilisés pour complexer les ions métalliques et possédant au moins deux groupes donneurs participant à la coordination de l'ion métallique, choisis parmi les carboxylates, les amines, les carbonyles, les alcools, les oximes, les phénols, les éthers, les thiols, les sulfonates et les amines aromatiques.

Les classes d'agents complexant préférés suivant la présente invention sont choisies parmi:
- les polyamines macrocycliques, avantageusement les dérivés de 1,5,9-triazacyclododécane de formule (II), le cyclen de formule (III) ou les dérivés de 1,4,7-triazacyclononane de formule (IV) 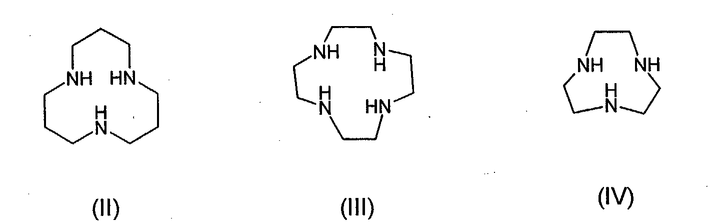
- les amino alcools, avantageusement la N,N-Bis(2-hydroxyéthyl)-éthylènediamine de formule (V), le 2-(2-amino éthylamino)-éthanol de formule (VI), ou la 1-(2-hydroxyéthyl)-pipérazine de formule (VII) 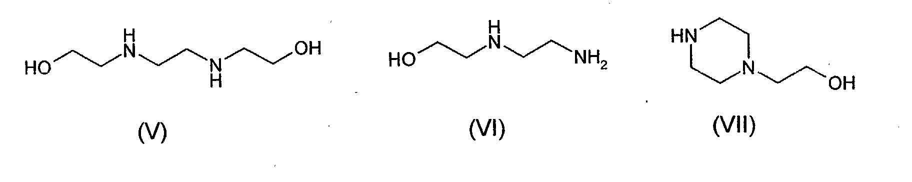
- les acides amino carboxyliques, avantageusement l'histidine de formule (VIII) ou l'acide N-(3,5-dimethoxybenzyl)-éthylènediamine-N,N',N'-triacétique de formule (IX) décrit dans la demande de brevet WO 9411338 ; 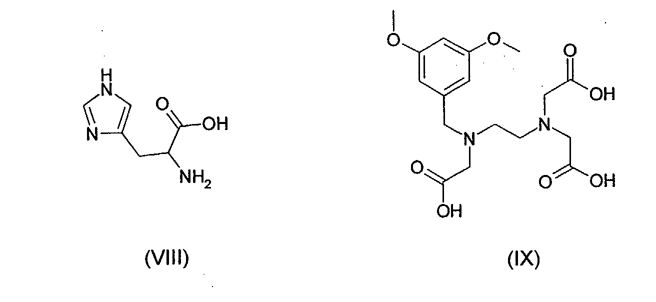
- les polyamines, avantageusement la tris-aminoethylamine (tren) de formule (X) ou le N-(3,4,5-trimethoxybenzyl)-N'{2-[2-(3,4,5-trimethoxybenzylamino)-éthylamino]éthyl}-éthane-1,2-diamine de formule (XI) ou le N,N',N"-tris-(3,4,5-trimethoxybenzyl) triéthylène tétramine de formule (XII) décrits dans la demande de brevet français n°96 07541 ; 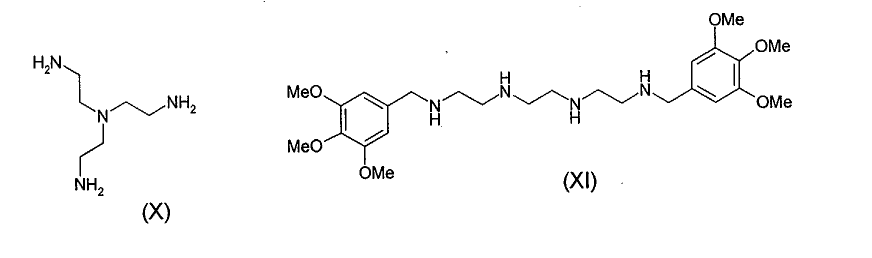 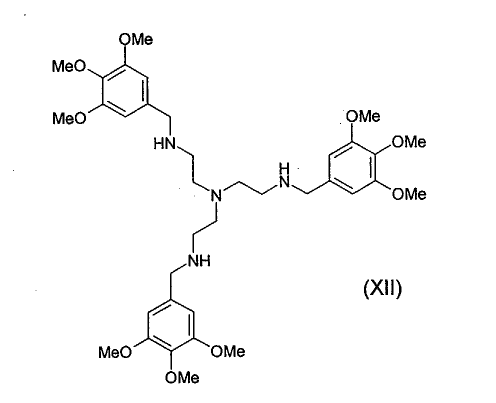
- les amines aromatiques, avantageusement la 2,2'-dipyridylamine de formule (XIII), le tris(2-benzimidazolylmethyl)amine de formule (XIV) et les dérivés de type 5-méthyle 3-*tert*-butyl-tris (pyrazolyle 1-borate) de formule (XV),

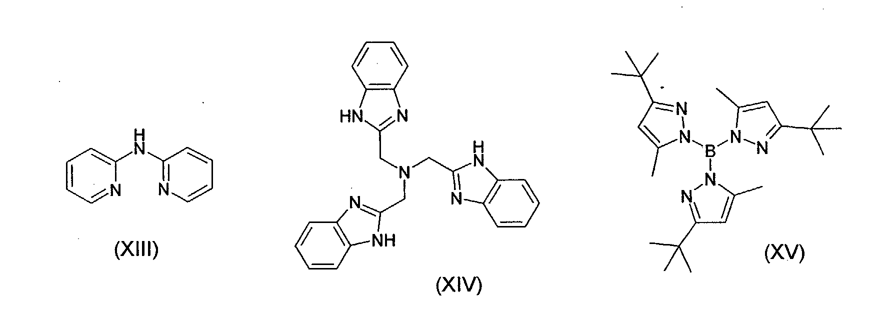

Bien entendu, selon l'invention, les complexes de métaux suivant l'invention peuvent être utilisés seuls ou en mélange et en toute proportion.

La quantité de complexe de métal utilisable selon l'invention, dépend bien évidemment de l'effet recherché et doit être en une quantité efficace pour favoriser la desquamation de la peau et/ou stimuler le renouvellement épidermique.

A titre d'exemple la quantité de complexe de métal suivant l'invention utilisable selon l'invention peut aller par exemple de 0,01% à 50% et de préférence de 0,1% à 10% du poids total de la composition.

Avantageusement, les complexes de métaux suivant l'invention sont utilisés dans des compositions cosmétiques (capillaires) en association avec des actifs antipelliculaires ; une desquamation du cuir chevelu permettant de renforcer l'efficacité d'agents antipelliculaire et rendre ainsi leur action plus efficace.

Avantageusement, les actifs antipelliculaires suivant l'invention sont choisis parmi le zinc pyrithione, la piroctone olamine, le disulfure de sélénium, le climbazole, l'acide undécylénique, le Kétoconazole, le ciclopyrox, l'octopyrox, un complexe formé par la tropolone et un sel métallique divalent, avantageusement de zinc, de cuivre, de calcium.

La composition dermatologique ou pharmaceutique utilisable selon l'invention peut être ingérée, injectée ou appliquée sur la peau (sur toute zone cutanée du corps), les cheveux, les ongles ou les muqueuses (buccale, jugale, gingivale, génitale, conjonctive). Selon le mode d'administration, la composition selon l'invention peut se présenter sous toutes les formes galéniques normalement utilisées, particulièrement en cosmétologie. Une composition préférée de l'invention est une composition cosmétique destinée à une application topique.

Pour une application topique sur la peau, la composition utilisable selon l'invention peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

La composition utilisable selon l'invention peut aussi être une composition pour soins capillaires, et notamment un shampooing, une lotion de mise en plis, une lotion traitante, une crème ou un gel coiffant, une composition de teintures (notamment teintures d'oxydation) éventuellement sous forme de shampooings colorants, des lotions restructurantes pour les cheveux, une composition de permanente (notamment une composition pour le premier temps d'une permanente), une composition permettant de réduire et/ou de stabiliser la chute naturelle des cheveux chez l'homme, avantageusement une lotion ou un gel, un shampooing antiparasitaire, etc.

Les quantités des différents constituants des compositions utilisables selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits après-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères.

Les compositions utilisables selon l'invention peuvent également consister en des préparations solides constituant des savons ou des pains de nettoyage.

Les compositions utilisables selon l'invention peuvent aussi être conditionnées sous forme d'une composition pour aérosol comprenant également un agent propulseur sous pression.

De façon connue, la composition selon l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse et/ou dans les sphérules lipidiques. En tout état de cause, ces adjuvants, ainsi que leurs proportions, seront choisis de manière à ne pas nuire aux propriétés recherchées du composé selon l'invention.

Lorsque la composition de l'invention est une émulsion, la proportion de la phase grasse peut aller de 5 à 80 % en poids, et de préférence de 5 à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine considéré. L'émulsionnant et le co-émulsionnant sont généralement présents, dans la composition, en une proportion allant de 0,3 à 30 % en poids, et de préférence de 0,5 à 20 % en poids par rapport au poids total de la composition. L'émulsion peut, en outre, contenir des vésicules lipidiques

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone et la fraction liquide du beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

Comme émulsionnants et coémulsionnants utilisables dans l'invention, on peut citer par exemple les émulsionnants H/E tels que les esters d'acide gras et de polyéthylène glycol, notamment le stéarate de PEG-100, et les esters d'acide gras et de glycérine tels que le stéarate de glycéryle, ainsi que les émulsionnants E/H tels que le poly(méthylcétyl)(diméthyl)méthylsiloxane oxyéthyléné disponible sous la dénomination commerciale ABIL WE09 auprès de la société Degussa Goldschmidt.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme charges qui peuvent être utilisées dans la composition de l'invention, on peut citer par exemple, outre les pigments, la poudre de silice ; le talc ; l'amidon réticulé par l'anhydride octénylsuccinique commercialisé par la société National Starch sous la dénomination DRY FLO PLUS (28-1160) ; les particules de polyamide et notamment celles vendues sous la dénomination ORGASOL par la société Atochem ; les poudres de polyéthylène ; les micro-sphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition ou de la préparation selon l'invention.

On peut également introduire dans la composition selon l'invention des filtres UVA et/ou UVB, choisis parmi les filtres organiques et les filtres minéraux éventuellement enrobés pour les rendre hydrophobes.

Selon un autre aspect, l'invention a pour objet une composition comprenant au moins l'association d'au moins un complexe de métal et d'au moins un autre agent choisi parmi les agents desquamants différents du dit complexe de métal, les agents hydratants, les agents dépigmentants ou propigmentants ; les agents anti-glycation ; les inhibiteurs de NO-synthase ; les agents permettant de réduire et/ou de stabiliser la chute naturelle des cheveux; les agents agissant sur les macromolécules dermiques ou épidermiques et/ou empêchant leur dégradation ; les agents stimulant la prolifération des fibroblastes et/ou des kératinocytes ou stimulant la différenciation des kératinocytes; les agents myorelaxants ; les agents anti-microbiens ; les agents tenseurs ; les agents anti-pollution et/ou anti-radicalaire ; les agents apaisants ; les actifs lipolytiques ou ayant une activité favorable, directe ou indirecte, sur la diminution du tissu adipeux ; les agents agissant sur la microcirculation ; les agents agissant sur le métabolisme énergétique des cellules ; et leurs mélanges.

### 1. Agents desquamants et hydratants

Par "agent desquamant", on entend tout composé capable d'agir :
- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de *Saphora japonica ;* le resvératrol ;
- soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut encore citer les composés aminosulfoniques et en particulier l'acide (N-2 hydroxyéthylpiperazine-N-2-éthane) sulfonique (HEPES) ; les dérivés de l'acide 2-oxothiazolidine-4-carboxylique (procystéine) ; les dérivés d'acides alpha aminés de type glycine (tels que décrits dans EP-0 852 949) ; le miel ; les dérivés de sucre tels que l'O-octanoyl-6-D-maltose et la N-acétyl glucosamine.

Par "agent hydratant", on entend :
- soit un composé agissant sur la fonction barrière, en vue de maintenir l'hydratation du stratum corneum, ou un composé occlusif. On peut citer les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;
- soit un composé augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et - ses dérivés, le chitosane, les oligo- et polysaccharides, les carbonates cycliques, l'acide N-lauroyl pyrrolidone carboxylique, et la N-α-benzoyl-L-arginine ;
- soit un composé activant les glandes sébacées tel que les dérivés stéroïdiens (dont la DHEA) et la vitamine D et ses dérivés.

Ces composés peuvent représenter de 0,001% à 30%, et de préférence de 0,01 à 20%, du poids total de la composition selon l'invention.

La composition selon la présente invention comprenant les agents hydratants cités ci-dessus est avantageusement destinée à la prévention ou au traitement du dessèchement de la peau et notamment des xéroses.

### 2. Agent dépigmentant ou pro-pigmentant

Les agents dépigmentants susceptibles d'être incorporés dans la composition selon la présente invention comprennent par exemple les composés suivants : l'acide kojique ; l'acide ellagique ; l'arbutine et ses dérivés tels que ceux décrits dans les demandes EP-895 779 et EP-524 109 ; l'hydroquinone ; les dérivés d'aminophénol tels que ceux décrits dans les demandes WO 99/10318 et WO 99/32077, et en particulier le N-cholestéryloxycarbonyl-para-aminophénol et le N-éthyloxycarbonyl-para-aminophénol ; les dérivés d'iminophénol, en particulier ceux décrits dans la demande WO 99/22707 ; l'acide L-2-oxothiazolidine-4-carboxylique ou procystéine, ainsi que ses sels et esters ; l'acide ascorbique et ses dérivés, notamment le glucoside d'ascorbyle ; et les extraits de plantes, en particulier de réglisse, de mûrier et de scutellaire, sans que cette liste soit limitative.

Comme agent pro-pigmentant, on peut citer l'extrait de pimprenelle (Sanguisorba officinalis) commercialisé par la société MARUZEN et les extraits de chrysanthème (Chrysanthemum morifolium).

La composition selon la présente invention comprenant les agents dépigmentants cités ci-dessus est avantageusement destinée à la prévention ou au traitement des hyperpigmentations, en particulier des taches pigmentaires liées au vieillissement de la peau.

De son côté, la composition renfermant les agents pro-pigmentants cités précédemment est de préférence destinée au traitement de la canitie.

### 3. Agent anti-glycation

Par "agent anti-glycation", on entend un composé prévenant et/ou diminuant la glycation des protéines de la peau, en particulier des protéines du derme telles que le collagène.

Des exemples d'agents anti-glycation sont les extraits végétaux de la famille des Ericaceae, tels qu'un extrait de myrtille (*Vaccinium angusfifollium*) ; l'ergothionéine et ses dérivés ; et les hydroxystilbènes et leurs dérivés, tels que le resvératrol et le 3,3', 5,5'-tétrahydroxystilbène. Ces agents anti-glycation sont décrits dans les demandes FR 99/16166, FR 00/08158, FR 99/09267 et FR 99/16168, respectivement. Le resvératrol est particulièrement préféré pour une utilisation dans cette invention.

La composition selon l'invention comprenant un agent anti-glycation tel que défini ci-dessus peut avantageusement être utilisée pour prévenir ou traiter les signes du vieillissement cutané, en particulier pour prévenir ou traiter la perte de tonicité et/ou d'élasticité de la peau.

### 4. Inhibiteur de NO-synthase

Des exemples d'inhibiteurs de NO-synthase convenant à une utilisation dans la présente invention comprennent notamment un extrait de végétal de l'espèce *Vitis vinifera* qui est notamment commercialisé par la société Euromed sous la dénomination Leucocyanidines de raisins extra, ou encore par la société Indena sous la dénomination Leucoselect®, ou enfin par la société Hansen sous la dénomination Extrait de marc de raisin ; un extrait de végétal de l'espèce *Olea europaea* qui est de préférence obtenu à partir de feuilles d'olivier et est notamment commercialisé par la société VINYALS sous forme d'extrait sec, ou par la société Biologia & Technologia sous la dénomination commerciale Eurol BT ; et un extrait d'un végétal de l'espèce *Gingko biloba* qui est de préférence un extrait aqueux sec de ce végétal vendu par la société Beaufour sous le nom commercial Ginkgo biloba extrait standard.

La composition selon l'invention comprenant un inhibiteur de NO-synthase tel que défini ci-dessus peut avantageusement être utilisée pour prévenir ou traiter les signes du vieillissement cutané et/ou les peaux sensibles.

### 5. les agents permettant de réduire et/ou de stabiliser la chute naturelle des cheveux

- les rétinoïdes, et en particulier le rétinol ;
- le soufre et les dérivés soufrés ;
- les sels de zinc tels que le lactate, le gluconate, le pidolate, le carboxylate, le salicylate et/ou le cystéate de zinc ;
- le chlorure de sélénium ;
- la vitamine B6 ou pyridoxine ;
- le mélange de capryloyl glycine, de sarcosine et d'extrait de cinnamomum zeylanicum commercialisé notamment par la société SEPPIC sous la dénomination commerciale Sepicontrol A5® ;
- un extrait de Laminaria saccharina commercialisé notamment par la société SECMA sous la dénomination commerciale Phlorogine® ;
- un extrait de Spiraea ulmaria commercialisé notamment par la société SILAB sous la dénomination commerciale Sebonormine® ;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- un extrait de Serenoa repens commercialisé notamment par la société EUROMED ;
- des extraits de plantes du genre Silybum ;
- des extraits végétaux contenant des sapogénines et en particulier les extraits de Dioscorées riches en diosgénine ou hécogénine ; et
- des extraits d'Eugenia caryophyllata contenant de l'eugénol et du glucoside d'eugényle.
- le 2,4-diamino-pyrimidine 3-oxyde ou 2,4-DPO décrit dans la demande de brevet WO 96/09048
- le 2,4-diamino-6-pipéridino pyrimidine 3-oxyde ou "Minoxidil" décrit dans les brevets US-4,139,619 et US-4,596,812

Ces composés sont par exemple présents dans la composition selon l'invention à hauteur de 0,001 à 10% en poids et, mieux, à hauteur de 0,01 à 5% en poids, par rapport au poids total de la composition.

### 6. Agent agissant sur les macromolécules dermiques ou épidermiques et/ou empêchant leur dégradation

Parmi les actifs stimulant les macromolécules du derme, on peut citer ceux qui agissent :
- soit sur la synthèse du collagène, tels que les extraits de Centella asiatica ; les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine®; et les hormones végétales telles que les auxines.
- soit sur la synthèse d'élastine, tels que l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ;
- soit sur la synthèse des glycosaminoglycanes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3®; et l'extrait de Saccharomyces cerevisiae disponible notamment auprès de la société SILAB sous la dénomination commerciale Firmalift® ou auprès de la société LSN sous la dénomination commerciale Cytovitin® ;
- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil® ;
- soit sur l'inhibition métalloprotéinases (MMP) telles que plus particulièrement les MMP 1, 2, 3, 9 . On peut citer : les rétinoïdes et dérivés, les isoflavonoïdes, lesoligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon ou de sauge ;
- soit sur l'inhibition des sérine protéases telles que l'élastase leucocytaire ou la cathepsine G. On peut citer: l'extrait peptidique de graines de légumineuse (Pisum sativum) commercialisé par la société LSN sous la dénomination commerciale Parelastyl® ; les héparinoïdes ; et les pseudodipeptides.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® ; et l'extrait de zooplancton Salina commercialisé par la société SEPORGA sous la dénomination commerciale GP4G®.

La composition selon l'invention renfermant un ou plusieurs des composés ci-dessus convient particulièrement bien à une utilisation dans la prévention ou le traitement des signes cutanés du vieillissement, en particulier de la perte de fermeté et/ou d'élasticité de la peau.

### 7. Agent modulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; et les hormones végétales telles que les giberrellines et les cytokinines.

Les agents stimulant la prolifération des kératinocytes, utilisables dans la composition selon l'invention, comprennent notamment les rétinoïdes tels que le rétinol et ses esters, dont le palmitate de rétinyle ; les extraits de tourteaux de noix commercialisés par la société GATTEFOSSE ; et les extraits de Solanum tuberosum commercialisés par la société SEDERMA.

Les agents stimulant la différenciation des kératinocytes comprennent par exemple les minéraux tels que le calcium ; l'extrait de lupin commercialisé par la société SILAB sous la dénomination commerciale Photopréventine®; le beta-sitosteryl sulfate de sodium commercialisé par la société SEPORGA sous la dénomination commerciale Phytocohésine® ; et l'extrait de maïs commercialisé par la société SOLABIA sous la dénomination commerciale Phytovityl®.

La composition selon l'invention comprenant ces composés est préférentiellement destinée à être utilisée pour prévenir ou traiter les signes cutanés du vieillissement.

### 8. Agent myorelaxant

Les agents myorelaxants utilisables dans la composition selon l'invention comprennent les inhibiteurs calciques tels que l'alvérine et ses sels, les ouvreurs de canaux chlore tel que le Diazepam, et les inhibiteurs de catécholamines et d'acétylcholine tels que l'hexapeptide argireline R commercialisé par la société LIPOTEC.

La composition selon l'invention comprenant ces composés est préférentiellement destinée à être utilisée pour prévenir ou traiter les signes cutanés du vieillissement et en particulier les rides.

### 9. Agent anti-microbien

Les agents antimicrobiens susceptibles d'être utilisés dans la composition selon l'invention peuvent notamment être choisis parmi le 2,4,4'-trichloro-2'-hydroxy diphényl éther (ou triclosan), le 3,4,4'-trichlorobanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, le 2,4,4'-trichloro-2'-hydroxy diphényl éther, le 3,4,4'-trichlorocarbanalide, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le brevet WO9318743, le farnesol, les phytosphingosines et leurs mélanges.

Les agents antimicrobiens préférés sont le triclosan, le phénoxyéthanol, l'octoxyglycérine, l'octanoylglycine, l'acide 10-hydroxy-2-décanoïque, le caprylyl glycol, le farnesol et l'acide azélaïque.

A titre d'exemple, l'agent antimicrobien peut être utilisé dans la composition selon l'invention en une quantité représentant de 0,1 à 20%, et de préférence de 0,1 à 10%, du poids total de la composition.

La composition renfermant l'agent anti-microbien convient particulièrement bien à une utilisation dans le traitement des peaux grasses à tendance acnéique, l'acné, ou les pellicules du cuir chevelu.

### 10. Agent tenseur

Par "agent tenseur", on entend un composé capable d'exercer une traction sur la peau, qui a pour effet d'estomper temporairement les irrégularités de la surface de la peau, telles que les rides et ridules.

Parmi les agents tenseurs utilisables dans la composition selon la présente invention, on peut citer notamment :
(1) les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique). De tels polymères siliconés greffés sont notamment vendus par la Société 3M sous les dénominations commerciales VS 80, VS 70 ou LO21,
(2) les protéines végétales de soja ou de blé, et/ou
(3) les silicates de sodium et magnésium (Laponites).

Les compositions selon l'invention comprenant les agents tenseurs ci-dessus sont avantageusement destinées au traitement des signes cutanés du vieillissement, en particulier des rides et ridules.

### 11. Agent anti-pollution ou anti-radicalaire

Par l'expression "agent anti-pollution", on entend tout composé capable de piéger l'ozone, les composés aromatiques mono- ou polycycliques tels que le benzopyrène et/ou les métaux lourds tels que le cobalt, le mercure, le cadmium et/ou le nickel. Par "agent anti-radicalaire", on entend tout composé capable de piéger les radicaux libres.

Comme agents piégeurs d'ozone utilisables dans la composition selon l'invention, on peut citer en particulier la vitamine C et ses dérivés dont le glucoside d'ascorbyle ; les phénols et polyphénols, en particulier les tannins, l'acide ellagique et l'acide tannique ; l'épigallocatéchine et les extraits naturels en contenant ; les extraits de feuille d'olivier ; les extraits de thé, en particulier de thé vert ; les anthocyanes ; les extraits de romarin ; les acides phénols, en particulier l'acide chlorogénique ; les stilbènes, en particulier le resvératrol ; les dérivés d'acides aminés soufrés, en particulier la S-carboxyméthylcystéine ; l'ergothionéine ; la N-acétylcystéine ; des caroténoïdes tels que la crocétine ; et des matières premières diverses comme le mélange d'arginine, ribonucléate d'histidine, mannitol, adénosinetriphosphate, pyridoxine, phénylalanine, tyrosine et ARN hydrolysé commercialisé par les Laboratoires Sérobiologiques sous la dénomination commerciale CPP LS 2633-12F®, la fraction hydrosoluble de maïs commercialisée par la société SOLABIA sous la dénomination commerciale Phytovityl®, le mélange d'extrait de fumeterre et d'extrait de citron commercialisé sous la dénomination Unicotrozon C-49® par la société Induchem, et le mélange d'extraits de ginseng, de pomme, de pêche, de blé et d'orge vendu par la société PROVITAL sous la dénomination commerciale Pronalen Bioprotect®.

Comme agents piégeurs de composés aromatiques mono- ou polycycliques utilisables dans la composition selon l'invention, on peut citer en particulier les tannins tels que l'acide ellagique ; les dérivés indoles, en particulier l'indol-3-carbinol ; les extraits de thé en particulier de thé vert, les extraits de Jacinthe d'eau ou eichornia crassipes ; et la fraction hydrosoluble de maïs commercialisée par la société SOLABIA sous la dénomination commerciale Phytovityl®.

Les agents anti-radicalaires utilisables dans la composition selon l'invention comprennent, outre certains agents anti-pollution mentionnés précédemment, la vitamine E et ses dérivés tels que l'acétate de tocophéryle ; les bioflavonoïdes ; le co-enzyme Q10 ou ubiquinone ; certaines enzymes comme la catalase, le superoxyde dismutase, la lactoperoxydase, le glutathion peroxydase et les quinones réductases ; le glutathion ; le benzylidène camphre ; les benzylcyclanones ; les naphtalénones substituées ; les pidolates ; le phytantriol ; le gamma-oryzanol ; les lignanes ; et la mélatonine.

### 12. Agents apaisants

Comme agents apaisants utilisables dans la composition selon l'invention, on peut citer les matières premières efficaces pour inhiber au moins l'une des enzymes choisies parmi les phospholipases, les lipooxygénases et les prostaglandines humaines synthétases, parmi lesquelles : les triterpènes pentacycliques et les extraits de plantes (ex : Glycyrrhiza glabra) en contenant comme l'acide β-glycyrrhétinique et ses sels et/ou ses dérivés (l'acide glyyrrhetique monoglucuronide, le stearyl glycyrrhétinate, l'acide 3- stéaroyloxy glycyrrhetique), l'acide ursolique et ses sels, l'acide oléanolique et ses sels, l'acide bétulinique et ses sels, l'extrait de Paeonia suffruticosa et / ou lactiflora, l'huile de calophilum, les sels de l'acide salicylique et en particulier le salicylate de zinc, les phycosaccharides (hydrolysed algin ou hydrolysed algin and zinc sulfate) de la société Codif, la phlorogine (laminaria saccharina) de Secma, l'huile de Canola, de Tamanu, de calophillum, l'β-bisabolol et les extraits de camomille, l'allantoine, le Sépivital EPC (diesterphosphorique de vitamine E et C) de Seppic, les huiles insaturées en oméga 3 telles que les huiles de rosier musca, de cassis, d'ecchium, de poisson, l'omega plancton (plancton extract) de Secma, le lipacide C8G ( capriloyl glycine) de Seppic, le Seppicalm VG (sodium palmitoylproline and nymphea alba) de Seppic, l'extrait d'epilobe, l'extrait du pygeum, le Soothex (extrait de boswellia serrata) de Quest, le phytoplenolin (extrait de centipeda cunnighami) de Bio-Botanica, l'Helioxine (extrait d'hélianthus annuus) de Silab, la Sensiline (linum usitatissimum) de Silab, les tocotrienols, les extraits de Cola nitida, le piperonal, l'extrait de clou de girofle, l'extrait d'épilobe (Epilobium Angustifolium) l'aloe vera, la bacocalmine (extrait de bacopa moniera) de Séderma, les phytostérols, la cortisone, l'hydrocortisone, l'indometacine la béta methasone.

La quantité des composés inhibiteurs de PLA2, Lox, PGHS-1 est bien entendu fonction de l'effet recherché et peut donc varier dans une large mesure.

Pour donner un ordre de grandeur, il peut-être utilisé en une quantité représentant de 0.001% à 10% du poids total de la composition et préférentiellement en une quantité représentant de 0.01% à 5% du poids total de la composition.

### 13. Les actifs lipolytiques ou ayant une activité favorable, directe ou indirecte, sur la diminution du tissu adipeux.

Parmi les dérivés susceptibles de favoriser la lipolyse on peut trouver :
1) les inhibiteurs de phosphodiestérase, tels que :
   - les dérivés xanthiques comme la caféine et ses dérivés, notamment les 1-hydroxyalkylxanthines décrites dans le document FR-A-2,617,401, la caféine citrate, la théophylline et ses dérivés, la théobromine, l'acéfylline, l'aminophylline, le chloroéthylthéophylline, le diprofylline, le diniprophylline, l'étamiphylline et ses dérivés, l'étofylline, la proxyphylline ;
   - les associations contenant des dérivés xanthiques, comme l'association de caféine et de silanol (dérivé méthylsilanetriol de caféine), et par exemple le produit commercialisé par la société Exsymol sous la dénomination caféisilane C ;
   - les composés d'origine naturelle contenant des bases xanthiques et notamment de la caféine, tels que les extraits de thé, de café, de guarana, de maté, de cola (*Cola Nitida*) et notamment l'extrait sec de fruit de guarana (*Paulina sorbilis*) contenant 8 à 10 % de caféine ;
   - l'éphédrine et ses dérivés qui peuvent notamment se retrouver à l'état naturel dans les plantes telles que le Ma Huang (Ephedra plant)
2) les extraits végétaux et les extraits d'origine marine, qui sont soit actifs sur les récepteurs à inhiber, tels que les β-2-bloqueurs, les NPY-bloqueurs (décrits dans le brevet EP 838217), soit inhibent la synthèse des récepteurs aux LDL ou VLDL, soit actifs pour stimuler les récepteurs β et les protéines G, conduisant à l'activation de l'adénylcyclase. Comme extraits végétaux de ce type, on peut citer par exemple :
   - le *Garcinia Cambogia,*
   - les extraits de *Bupleurum chinensis*,
   - les extraits de lierre grimpant (*Hedera Helix*), d'arnica *(Arnica Montana L*), de romarin (*Rosmarinus officinalis N*), de souci (*Calendula officinalis*), de sauge (*Salvia officinalis L*), de ginseng *(Panax ginseng*), de millepertuis (*Byperycum Perforatum*), de fragon *(Ruscus aculeatus L*), d'ulmaire (*Filipendula ulmaria L*), d'orthosiphon (*Orthosiphon Stamincus Benth*), de bouleau (*Betula alba*), de cécropia et d'arganier.
   - les extraits de ginkgo biloba,
   - les extraits de prêle,
   - les extraits d'escine,
   - les extraits de cangzhu,
   - les extraits de *chrysanthellum indicum*,
   - les extraits de dioscorés riches en diosgénine ou la diosgénine ou hécogénine pure et leurs dérivés.
   - les extraits des plantes du genre *Armeniacea, Atractylodis Platicodon, Sinom-menum, Pharbitidis, Flemingia,*
   - les extraits de *Coleus* tels que *C. Forskohlii, C. blumei, C. esquirolii, C. scutellaroïdes*, *C. xanthantus* et *C. Barbatus*, tel que l'extrait de racine de *Coleus Barbatus* contenant 60 % de forskoline,
   - les extraits de Ballote,
   - les extraits de *Guioa,* de *Davallia,* de *Terminalia,* de *Barringtonia*, de *Tréma,* d'*Antirobia*.

   Comme extrait d'origine marine on peut citer les extraits d'algues ou de phytopancton, tels que le rhodystérol ou l'extrait de *Laminaria Digitata* commercialisé sous la dénomination PHYCO R 75 par la société Secma, l'algue skeletonema décrite dans le brevet FR 2 782 921 ou les diatomées décrites dans le brevet FR 2774292.
3) les peptides ou protéines
   - les peptides dérivés de l'hormone parathyroidienne tels que décrits dans les brevets FR 2 788058 et FR 2781231 de Séderma ou les peptides décrits dans le document FR 2 786 693 voire tout autre peptide ayant des propriétés lipolytiques,
   - les protamines et leurs dérivés tels que ceux décrits dans le document FR-A-2,758,724.

La quantité d'actif(s) lipolytique(s) peut varier dans une large mesure et dépend de la nature de ou des actifs utilisés. De manière générale, le ou les actifs amincissants sont présents en une concentration allant de 0,001 à 20 % et de préférence de 0,1 à 10 % en poids par rapport au poids total de la composition.

### 14. Les agents agissant sur la microcirculation

Les actifs agissant sur la microcirculation (vasoprotecteurs ou vasodilatateurs), se trouvent notamment parmi les flavonoïdes, les ruscogénines, les esculosides, l'escine extraite du marron d'Inde, les nicotinates, l'héperidine méthyl chalcone, le petit houx, les huiles essentielles de lavande ou de romarin , les extraits de *Ammi Visnaga*.

La quantité de ces actifs peut varier dans une large mesure. De manière générale, ces actifs sont présents en une concentration allant de 0,01 à 15 % et de préférence de 0,05 à 10 % en poids par rapport au poids total de la composition.

### 15. Les agents agissant sur le métabolisme énergétique des cellules

Par cette expression, on entend les actifs agissant sur le métabolisme énergétique cutané tel que, par exemple, et de façon non limitative, la synthèse d'ATP, ainsi que ceux qui interviennent sur la chaîne respiratoire de la cellule ou sur les réserves énergétiques. On peut citer à ce titre le Coenzyme Q10 (ubiquinone), le cytochrome C, la créatine ou encore la phosphocréatine.

Comme indiqué précédemment, la composition selon l'invention peut également renfermer des filtres UVA et/ou UVB, sous forme de composés organiques ou inorganiques, ces derniers étant éventuellement enrobés pour les rendre hydrophobes.

Les filtres organiques peuvent notamment être choisis parmi : les anthranilates, en particulier l'anthranilate de menthyle ; les benzophénones, en particulier la benzophénone-1, la benzophénone-3, la benzophénone-5, la benzophénone-6, la benzophénone-8, la benzophénone-9, la benzophénone-12, et préférentiellement la Benzophénone-2 (Oxybenzone), ou la Benzophénone-4 (Uvinul MS40 disponible chez B.A.S.F.) ; les benzylidènes-camphres, en particulier le 3-benzylidène-camphre, l'acide benzylidènecampho-sulfonique, le benzalkoniumméthosulfate de camphre, le polyacrylamidométhylbenzylidène camphre, l'acide téréphthalylidène di-camphre sulfonique, et préférentiellement le 4-méthylbenzylidène camphre (Eusolex 6300 disponible chez Merck) ; les benzimidazoles, en particulier le benzimidazilate (Neo Heliopan AP disponible chez Haarmann et Reimer), ou l'acide phénylbenzimidazole sulfonique (Eusolex 232 disponible chez Merck) ; les benzotriazoles, en particulier le drométrizole trisiloxane, ou le méthylène bis-benzotriazolyltétraméthylbutylphénol (Tinosorb M disponible chez Ciba) ; les cinnamates, en particulier le cinoxate, le DEA méthoxycinnamate, le méthylcinnamate de diisopropyle, le glycéryl éthylhexanoate de diméthoxycinnamate, le méthoxycinnamate d'isopropyle, le cinnamate d'isoamyle, et préférentiellement l'éthocrylène (Uvinul N35 disponible chez B.A.S.F.), l'octylméthoxycinnamate (Parsol MCX disponible chez Hoffmann La Roche), ou l'octocrylène (Uvinul 539 disponible chez B.A.S.F.) ; les dibenzoylméthanes, en particulier le butyl méthoxydibenzoylméthane (Parsol 1789) ; les imidazolines, en particulier l'éthylhexyl diméthoxybenzylidène dioxoimidazoline ; les PABA, en particulier l'éthyl Dihydroxypropyl PABA, l'éthylhexyldiméthyl PABA, le glycéryl PABA, le PABA, le PEG-25 PABA, et préférentiellement la diéthylhexylbutamido-triazone (Uvasorb HEB disponible chez 3V Sigma), l'éthylhexyltriazone (Uvinul T150 disponible chez B.A.S.F.), ou l'éthyl PABA (benzocaïne) ; les salicylates, en particulier le salicyclate de dipropylèneglycol, le salicylate d'éthylhexyle, l'homosalate, ou le TEA salicylate ; les triazines, en particulier l'anisotriazine (Tinosorb S disponible chez Ciba) ; le drometrizole trisiloxane.

Les filtres inorganiques sont de préférence constitués d'oxyde de zinc et/ou de dioxyde de titane, de préférence de taille nanométrique, éventuellement enrobés d'alumine et/ou d'acide stéarique.

L'invention est illustrée plus en détail dans les exemples suivants. Ces exemples ne sauraient limiter en aucune façon la portée de l'invention.

### Exemple 1: Exemple de complexes métalliques

### Synthèse du complexe de zinc de l'acide N,N'-Bis(dibenzyl)ethylenediamine N,N'-diacétique.

0.61 g d'acétate de zinc dihydraté sont solubilisés dans 5 ml d'eau.

On ajoute 1g d'acide N,N'-Bis(dibenzyl)ethylenediamine N,N'-diacétique (L1, préparé comme décrit dans la demande WO9411338) en solution dans 10 ml d'ammoniaque à 1%.

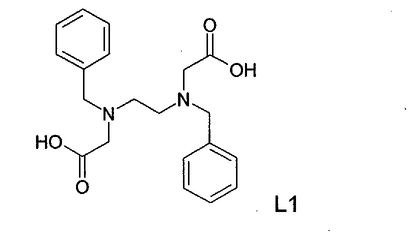

Le mélange est agité pendant une heure à température ambiante.

Le précipité blanc est filtré sur verre fritté et lavé à l'eau puis séché sous vide sur P₂O₅.

On obtient 0.86 g de poudre blanche correspondant au complexe Zn-L1.

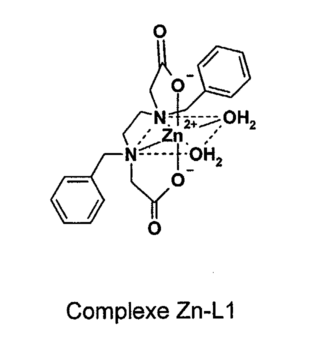

| Analyse élémentaire | | | | | |
|---|---|---|---|---|---|
| | C | H | N | O | Zn |
| Attendu | 52.60 | 5.70 | 6.10 | 21.00 | 14.50 |
| Trouvé | 53.35 | 5.59 | 5.71 | 21.87 | 13.30 |

Alternativement, les complexes selon l'invention peuvent être préparés extemporanément en mélangeant un équivalent molaire de sel métallique avec un à deux équivalents molaires de ligand en solution dans un solvant approprié.

### Synthèse du

Ainsi, une solution 1 mM de complexe de zinc de la N,N-Bis(2-hydroxyethyl) éthylènediamine (L2) est préparée en mélangeant 3,7 mg de perchlorate de zinc Zn(ClO₄)₂ (hexahydrate) et 3.0 mg de ligand L2 dans 10 ml d'eau, puis en agitant 30 min à température ambiante.

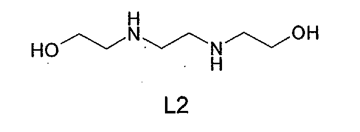

De même, une solution 1 mM de complexe de zinc de la tris(2-benzimidazolylmethyl) amine (L3) est préparé en mélangeant un équivalent molaire de perchlorate de zinc Zn(ClO₄)₂ (hexahydrate) et de ligand L3 dans 10 ml d'un mélange eau acétonitrile 1 :1, puis en agitant 30 min à température ambiante.

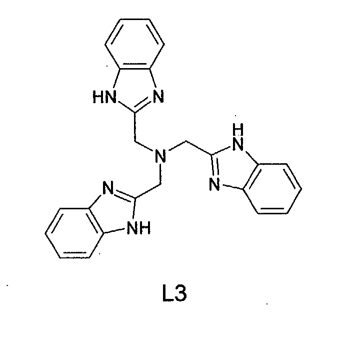

Enfin, selon le même procédé en partant de chlorure de colbalt et de N-(3,4,5-triméthoxybenzyl)-N,N'-bis-[2-(3,4,5-trimethoxybenzylamino)-éthyl]-éthane-1,2-diamine (L4, préparé selon la demande de brevet français n°96 07541 ), on obtient une solution de complexe de cobalt du ligand L4.

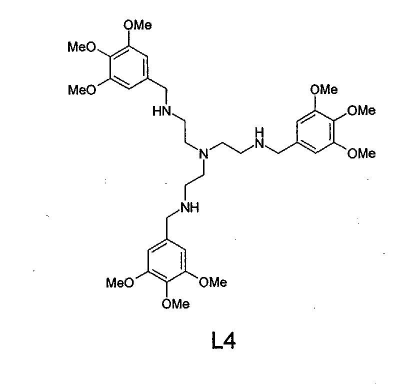

### Exemple 2:

### Mise en oeuvre des propriétés hydrolytiques du complexe Co-L4

Dans une cuvette UV, on ajoute successivement :
- 2,6 ml de tampon tris 50 mM à pH 8 ,
- 300 µl d'une solution 10 mM en complexe Co-L4 dans l'eau et,
- 100 µl d'une solution d'acétate de p-nitrophénol 10 mM dans l'acétonitrile.

On suit alors l'augmentation de la densité optique à 410 nm à 37°C en fonction du temps pendant 1 heure à l'aide d'un spectrophotomètre UV visible.

On observe un rapport de 2.1 entre la pente de la droite obtenue et celle de la droite obtenue dans une expérience témoin sans complexe, qui mesure l'hydrolyse spontanée du substrat.

### Exemple 3: Compositions

On prépare les compositions suivantes de manière classique pour l'homme du métier.

### Crème prodesquamante pour le visage

- Complexe Zn-L1 2,00 %
- Stéarate de sodium 3,00 %
- Huile de vaseline 6,00 %
- Alkyl paraben 0,05 %
- Sorbate de potassium 10,00 %
- Alcool stéarylique 1,00 %
- Parfum 1,00 %
- Eau qsp 100,00 %

### Crème prodesquamante pour le corps

- Complexe Co-L2 5,0 %
- Huile de jojoba 13,0 %
- Cire de sipol 6,0 %
- Palmitate d'isopropyle 2,0 %
- Glycérol 15,0 %
- Alkyl paraben 0,5 %
- Parfum 1,0 %
- Eau qsp 100,0 %

### Crème de soin prodesquamante

- Complexe Zn-L2 1 %
- Polyéthylène glycol 50 oxyéthyléné 3 %
- Mono diglycéryl stéarate 3 %
- Huile de vaseline 24 %
- Acool cétylique 5 %
- Eau qsp 100 %

### Crème de soin desquamante pour le corps

- Complexe Cu-L1 0,5 %
- Cire de sipol 6,0 %
- Glycéryl monostéarate 1,5 %
- Stéarate de sodium 0,8 %
- Huile de vaseline 6,0 %
- Palmitate d'isopropyle 2,0 %
- Glycérol 15,0 %
- Parfum 0,3 %
- Eau qsp 100,0 %

### Crème de soin prodesquamante

- Complexe Zr-L1 0,50 %
- Huile de jojoba 13,00 %
- Alkyl paraben 0,05 %
- Sorbate de potassium 0,30 %
- Cyclopenta diméthylsiloxane 10,00 %
- Alcool stéarylique 1,00 %
- Acide stéarique 4,00 %
- Stéarate de polyéthylène glycol 3,00 %
- Vitamine E 1,00 %
- Glycérol 3,00 %
- Eau qsp 100,00 %

## Revendications

1. Utilisation cosmétique d'au moins un complexe de métal, dans une composition cosmétique comprenant un milieu physiologiquement acceptable, en tant qu'agent cosmétique destiné à favoriser la desquamation de la peau et/ou à stimuler le renouvellement épidermique, le dit complexe de métal ayant une capacité à accélérer d'au moins un facteur 2 l'hydrolyse en solution aqueuse à pH neutre ou légèrement alcalin ou dans un mélange eau-acétonitrile ou eau-acétone, d'un substrat choisi parmi le phosphate de p-nitro phénol, l'acétate de p-nitro phénol, l'acétate de p-nitro aniline.

2. Utilisation selon la revendication 1, **caractérisée en ce que** le complexe de métal répond à la formule (I) suivante:
(I) Lₚ - Mⁿ⁺
dans laquelle,
- M représente un métal choisi par le zinc, le cobalt, le cuivre, le fer, le lanthane, le palladium, le manganèse, le molybdène, l'europium, le cérium ou le zirconium;
- n désigne un nombre choisi parmi 2, 3 ou 4 ;
- L représente un agent complexant choisi parmi un bidentate, un tridentate, un tétradentate, un pentadentate ou hexadentate ;
- p désigne un nombre choisi parmi 1, 2 ou 3.

3. Utilisation selon la revendication 2, **caractérisée en ce que** l'agent complexant possède au moins deux groupes donneurs participant à la coordination de l'ion métallique, choisis parmi les carboxylates, les amines, les carbonyles, les alcools, les oximes, les phénols, les ethers, les thiols, les sulfonates et les amines aromatiques.

4. Utilisation selon l'une quelconques des revendications 2 ou 3, **caractérisé en ce que** l'agent complexant est choisi parmi les polyamines macrocycliques, les amino-alcools, les acides amino-carboxyliques, les polyamines, les amines aromatiques.

5. Utilisation selon l'une quelconques des revendications 2 à 4, **caractérisé en ce que** l'agent complexant est choisi parmi
- les dérivés de 1,5,9-triazacyclododécane de formule (II), le cyclen de formule (III) les dérivés de 1,4,7-triazacyclononane de formule (IV) 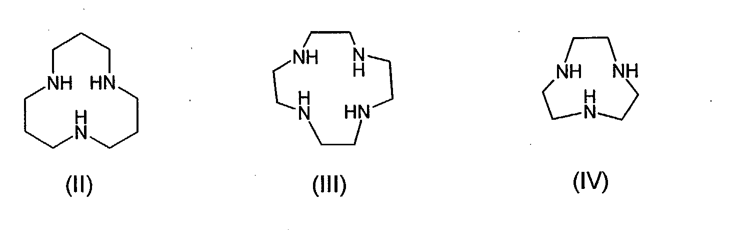
- la N,N-Bis(2-hydroxyéthyl)-éthylènediamine de formule (V), le 2-(2-amino éthylamino)-éthanol de formule (VI), la 1-(2-hydroxyéthyl)-pipérazine de formule (VII) 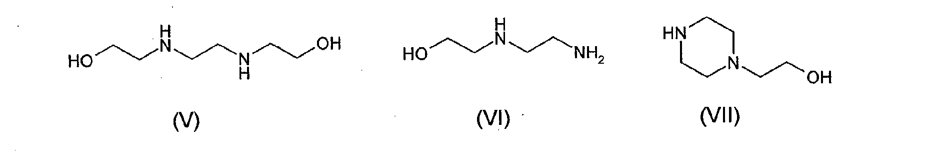
- l'histidine de formule (VIII), l'acide N-(3,5-dimethoxybenzyl)-éthylènediamine-N,N',N'-triacétique de formule (IX); 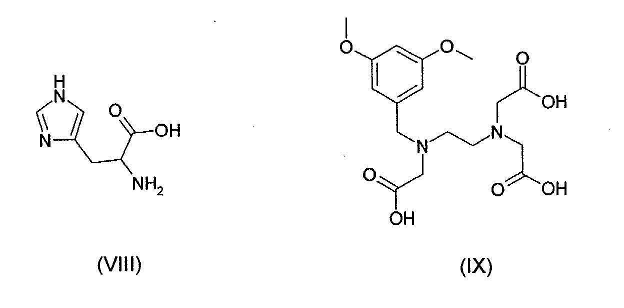
- la tris-aminoethylamine (tren) de formule (X) le N-(3,4,5-trimethoxybenzyl)-N'{2-[2-(3,4,5-trimethoxybenzylamino)-éthylamino]éthyl}-éthane-1,2-diamine de formule (XI) ou le N,N',N"-tris-(3,4,5-trimethoxybenzyl) triethylene tetramine de formule (XII) ; 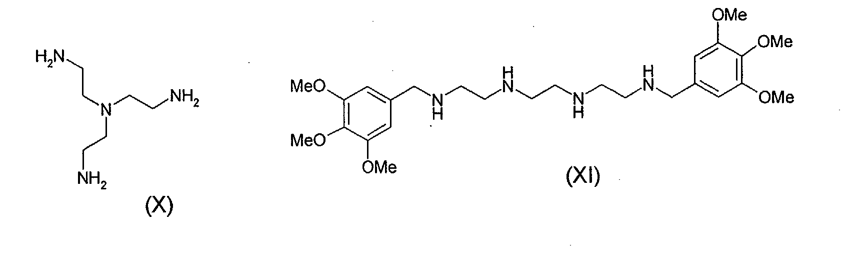 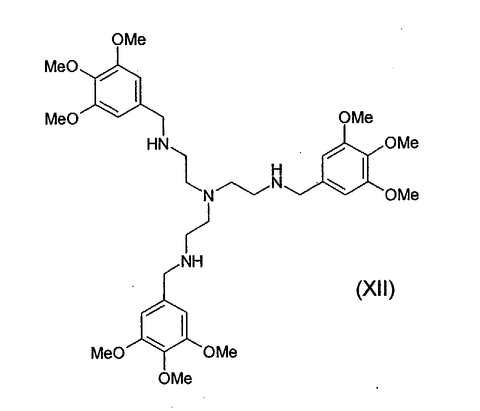
- la 2,2'-dipyridylamine de formule (XIII), le tris(2-benzimidazolylmethyl)amine de formule (XIV) et les dérivés de type 5-methyle 3-*tert*-butyl-tris (pyrazolyle 1-borate) de formule (XV),

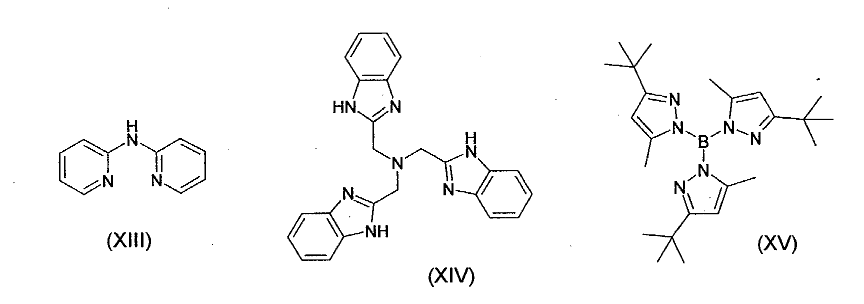

6. Utilisation cosmétique d'un complexe de métal tel que défini dans l'une quelconque des revendications précédentes, dans une composition cosmétique, en tant qu'agent pour prévenir ou traiter la peau sèche et/ou les signes cutanés du vieillissement et/ou la pigmentation de la peau et/ou la peau grasse à tendance acnéique.

7. Utilisation cosmétique d'un complexe de métal tel que défini dans l'une quelconque des revendications précédentes, dans une composition cosmétique en association avec un agent antipelliculaire, pour renforcer l'efficacité du dit agent antipelliculaire.

8. Utilisation suivant la revendication précédente, **caractérisée en ce que** l'actif antipelliculaire est le zinc pyrithione, la piroctone olamine, le disulfure de sélénium, le climbazole, l'acide undécylénique, le Kétoconazole, le ciclopyrox, l'octopyrox, un complexe formé par la tropolone et un sel métallique divalent, avantageusement de zinc, de cuivre, de calcium.

9. Procédé de traitement cosmétique de la peau sèche et/ou des signes cutanés du vieillissement et/ou de la peau grasse à tendance acnéique, comprenant l'application topique sur la peau d'une composition comprenant, dans un milieu physiologiquement acceptable, au moins un complexe de métal décrit dans l'une quelconque des revendications 1 à 5.

10. Procédé de traitement cosmétique pour diminuer la pigmentation de la peau comprenant l'application topique sur la peau d'une composition comprenant, dans un milieu physiologiquement acceptable et au moins un complexe de métal décrit dans la revendication 5.

11. Procédé selon l'une quelconque des revendications 9 et 10, **caractérisé en ce que** ladite composition renferme de 0,01% à 50%, de préférence de 0,1% à 10% en poids de complexe de métal, par rapport au poids total de la composition.

12. Utilisation d'au moins un complexe de métal tel que défini dans l'une quelconque des revendications 1 à 5, pour la préparation d'une composition destinée à prévenir ou traiter les désordres cutanés liés aux hyperkératoses.

13. Utilisation selon la revendication 12, **caractérisée en ce que** les désordres liés aùx hyperkératoses sont choisis parmi : le parapsoriasis, le psoriasis, l'ichtyose et le lichen.

14. Composition renfermant, dans un milieu physiologiquement acceptable, au moins un complexe de métal tel que défini dans l'une quelconque des revendications 1 à 5, et au moins un composé choisi parmi :
- les agents desquamants différents du dit complexe de métal,
- les agents hydratants en une quantité représentant de 0,001% à 30% du poids total de la composition ;
- les agents dépigmentants ou propigmentants ;
- les agents anti-glycation ;
- les inhibiteurs de NO-synthase ;
- les agents permettant de réduire et/ou de stabiliser la chute naturelle des cheveux;
- les agents agissant sur les macromolécules dermiques ou épidermiques et/ou empêchant leur dégradation ;
- les agents stimulant la prolifération des fibroblastes et/ou des kératinocytes ou stimulant la différenciation des kératinocytes ;
- les agents myorelaxants ;
- les agents anti-microbiens en une quantité représentant de 0,1 à 20% du poids total de la composition ;
- les agents tenseurs ;
- les agents anti-pollution et/ou anti-radicalaire ;
- les agents apaisants ;
- les actifs lipolytiques ou ayant une activité favorable, directe ou indirecte, sur la diminution du tissu adipeux ;
- les agents agissant sur la microcirculation ;
- les agents agissant sur le métabolisme énergétique des cellules ;
- et leurs mélanges.
